# EUROPEAN PATENT APPLICATION

(11) **EP 2 719 353 A1**
(43) Date of publication of application: **16.04.2014**
(21) Application number: 12796647.1
(22) Date of filing: 05.06.2012
(51) Int. Cl.: A61B 19/00, A61B 17/56

(54) **METHOD FOR MANUFACTURING REGISTRATION TEMPLATE**

(30) Priority: 06.06.2011 JP 2011126261
(71) Applicant: Matsumoto, Nozomu, Fukuoka 812-8582 (JP); Ono & Co., Ltd., Tokyo 158-0083 (JP)
(72) Inventor: ONO, Hidenori, Tokyo 158-0083 (JP); SUGIYAMA, Hisayuki, Tokyo 158-0083 (JP)
(74) Representative: Müller-Boré & Partner Patentanwälte PartG mbB
(86) International application number: PCT/JP2012/064860
(87) International publication number: WO 2012/169642

(57) **Abstract**

A method for manufacturing a registration template for use in medical navigation system-guided surgery, comprising: producing a registration template having a surface, which precisely surface-bonds to a surface of a bone at a surgical target site of a patient, and having three or more registration points, from stereoscopic surface data ensuring precise surface bonding to the surface of the bone at the surgical target site of the patient, based on stereoscopic surface data created from tomography information on the bone of the surgical target site of the patient; and providing a pedestal (4) for mounting optical trackingballs (7), which are used for three-dimensional position detection for a medical navigation system, at the center of the registration points. By using the template having curves surfaces which three-dimensionally precisely and intimately contact the bone surface of the patient, high-precision registration can be performed, and an operator's man-hours immediately before surgery in an operating room can be reduced to shorten the surgery time.

## Description

### Technical Field

This invention relates to a method for manufacturing a registration template in a medical navigation system. More specifically, the present invention relates to a method for manufacturing a registration template which has curved surfaces conforming precisely to the curved surfaces or the like of a bone at a target site for surgery (may be called a surgical target site) in a patient. In performing surgery, the method involves determining a position at which the curved surfaces of the registration template closely contact and fit into the site of surgery in the patient fixed to an operating table; prestoring in the navigation system the relative coordinates of three or more registration points provided in the registration template and balls for a three-dimensional position detecting light reflector (may be referred to hereinafter as an optical tracking antenna in the present invention), which is mounted on the template, at the position of the close contact and fitting; and making registration of the three-dimensional coordinates of the balls, thereby automatically navigating the position of a surgical tool, whichmoves during surgery, on a three-dimensional stereoscopic image of the surgical target site. The registration template is used for this navigation.

### Background Art

The medical navigation system is a medical instrument, or a combination of medical instruments, intended to display the positional relationship between a surgical target site in a patient during surgery and a surgical tool such as a surgical knife, a drill or a robot hand.

The navigation system is utilized in craniocervical surgery, neurosurgery, otorhinolaryngology, orthopedics, plastic surgery, oral surgery, aesthetic surgery or the like, and is expected to develop further in the future.

Setup of the navigation system is divided into 4 steps, i.e., photographing of a surgical target site in a patient, capture of photographed images, registration, and navigation.

The registration step is to automatically align the positional relationship between the image during navigation and the actual surgical target site of the patient, and is performed after the patient assumes a posture for surgery. Thus, the registration step is normally carried out in an operating room, and is desirably performed in the shortest time possible from the point of view of reducing a burden on the patient and the preoperative man-hours of the operator. Moreover, the precision of registration directly affects the accuracy of the position of the surgical tool during navigation, and presents a very important problem in the medical navigation system.

As a prior art method for registration in the medical navigation system, for example, point registration is known which involves alignment of the actual surgical target site with the image data of the medical navigation system based on markers pasted to the skin of the patient before picture taking. Surface registration is also available in which more points on the surface of the skin are inputted randomly based on the point registration to combine three-dimensional shapes (see, for example, Non-Patent Document 1).

However, the pasting of the markers is usually performed on the day before surgery, thus requiring restrictions on the patient' s behaviors. Moreover, the surface portion of the skin in the patient is used as a reference, but the surface of the skin or the like moves or deforms. Thus, precise registration is difficult to perform.

As a conventional technology, a technology is disclosed in which a probe using an articulated arm is brought into contact with the surface of a patient's skin to obtain position information (see, for example, Patent Document 1).

This technology, however, is also defective in that registration on the skin surface of the patient is poor in precision and that if a deviation occurs between the surgical target site in the patient and the image on the navigation system for a cause ascribed to the system or the patient during surgery, re-registration using an already incised skin is impossible.

As another conventional technology, a method comprising directly applying metallic markers or the like to a hard part, such as a patient's bone, is disclosed (see, for example, Patent Document 2). This technology is defective, however, in that in order to photograph the markers, photography must be done again, and a burden such as incision or perforation is imposed on the patient before surgery.

### [Prior Art Documents]

### [Patent Documents]

[Patent Document 1] JP-A-2001-238895
[Patent Document 2] JP-A-2006-81569

### [Non-Patent Document]

[Non-Patent Document 1] Journal of Japanese Society of Radiological Technology Kinki Branch, Vol. 10, No. 1

### Disclosure of the Invention

### [Problems to be solved by the invention]

The present invention has been accomplished under these circumstances. It is an obj ect of the present invention to enable high-precision registration in a medical navigation system by use of a registration template (may hereinafter be described simply as a template in the present invention) having curves surfaces, which three-dimensionally precisely and intimately contact the surface of a patient' s bone, and reduce an operator's man-hours immediately before surgery in an operating room to shorten the surgery time.

### [Means for solving the problems]

The present inventors conducted in-depth studies in an attempt to solve the above-mentioned problems. As a result, the inventors have found the following facts: Use is made of a registration template having precise three-dimensional curved surfaces which surface-bond to the stereoscopic surface of a natural bone based on three-dimensional image data on a patient who undergoes surgery. Moreover, the relative positions of registration points and a three-dimensional position detecting reflector which is used in a navigation system and mounted on a pedestal provided in the template are prestored in the navigation system. By so doing, precise registration in a medical navigation system can be achieved in a very short time. Besides, there is no need any more for a surgical operation or CT only for the purpose of registration before the intended surgery. Thus, no physical or mental burden is imposed on the patient. As noted here, the use of the registration template produced based on image information obtained by image photographing during medical examination enables registration to be achieved by a simple and prompt surface bonding procedure. Based on these findings, the present invention has been accomplished.

That is, the present invention provides the following:
(1) A method for manufacturing a registration template having three or more registration points for use in medical navigation system-guided surgery, the method comprising: producing a template having a surface, which precisely surface-bonds to a surface of a bone at a surgical target site of a patient, by a production method selected from among stereoscopic shaping, cutting, and casting, from stereoscopic surface data ensuring precise surface bonding to the surface of the bone at the surgical target site of the patient, based on stereoscopic surface data created from tomography information on the bone at the surgical target site of the patient; and providing a pedestal for mounting optical tracking balls, which are used for three-dimensional position detection for a medical navigation system, at the center of the registration points of the template.
(2) The method for manufacturing a registration template according to (1), further comprising: producing a template having a mounting portion for the pedestal for mounting the optical tracking balls, which are used for three-dimensional position detection for the medical navigation system, at a location corresponding to the center of the three or more registration points for use in the medical navigation system, the location being on a surface on a side opposite to the surface precisely surface-bonding to the surface of the bone at the surgical target site; and providing the pedestal for mounting the optical tracking balls, on the mounting portion for the pedestal for mounting the optical tracking balls.
(3) The method for manufacturing a registration template according to (2), wherein the mounting portion for the pedestal for mounting the optical tracking balls has a columnar shape.
(4) The method for manufacturing a registration template according to any one of (1) to (3), wherein the registration points are embossed marks.
(5) The method for manufacturing a registration template according to any one of (1) to (3), wherein the registration points are guide holes.
(6) The method for manufacturing a registration template according to any one of (1) to (5), further comprising producing the template, which has the surface precisely surface-bonding to the surface of the bone at the surgical target site, by stereolithography.
(7) The method for manufacturing a registration template according to any one of (1) to (6), further comprising: producing the template having the surface, which precisely surface-bonds to the surface of the bone at the surgical target site of the patient, from the stereoscopic surface data ensuring precise surface bonding to the surface of the bone at the surgical target site of the patient, based on the stereoscopic surface data created from the tomography information on the bone at the surgical target site of the patient; and processing the data to produce a model of the surgical target site of the patient.
(8) A method for manufacturing a registration template having three or more registration points for use in medical navigation system-guided surgery, and having a surface precisely surface-bonding to a surface of a bone at a surgical target site, the method comprising: producing a template, in which the surface precisely surface-bonding to the surface of the bone at the surgical target site has not been formed, but a mounting portion for a pedestal for mounting optical tracking balls for use in three-dimensional position detection for a medical navigation system has been provided at the center of the registration points of the template, by a production method selected from stereoscopic shaping and cutting; producing a model of the surgical target site based on three-dimensional data created from tomography information on the bone at the surgical target site of a patient and position information on three or more predetermined registration points; pressing the surface of the bone at the surgical target site in the model, as an embossing die, against a mold material of predetermined dimensions, and pressing the mold material having a surface shape of the model of the surgical target site against a surface of the template, in which the surface precisely surface-bonding to the surface of the bone at the surgical target site has not been formed and is to be formed, to transfer the surface shape of the model of the surgical target site to the surface of the template, thereby producing the template having the surface shape transferred thereto; producing a mold by use of the resulting template as a mold producing master; and producing the template by casting using the mold.
(9) A method for manufacturing a registration template having three or more registration points for use in medical navigation system-guided surgery, and having a surface precisely surface-bonding to a surface of a bone at a surgical target site, the method comprising: producing a model of a surgical target site of a patient by stereoscopic shaping based on three-dimensional image data created from tomography information on the surgical target site of the patient; scanning a surface of a site in the model, corresponding to the surgical target site, by a noncontact laser scanning device to obtain stereoscopic surface data ensuring precise surface bonding to the surface of the site in the model corresponding to the surgical target site; loading the resulting stereoscopic surface data onto editing software; duplicating and moving the stereoscopic surface data loaded onto the editing software for conversion into a plate form; imparting data on three or more predetermined registration points to the plate form; and further providing a mounting portion for a pedestal for mounting optical tracking balls for use in three-dimensional position detection for a medical navigation system, at the center of the plate form by use of a shape generating function of the editing software, thereby producing a template having a surface precisely surface-bonding to the surface of a bone at the surgical target site.
(10) The method for manufacturing a registration template according to any one of (1) to (9), wherein the registration template is transparent.
(11) The method for manufacturing a registration template according to any one of (1) to (10), wherein the registration template has at least some of concave and convex surfaces of a shape identical with a shape of concave and convex surfaces of the bone at the surgical target site, on a surface opposite to the surface precisely surface-bonding to the surface of the bone at the surgical target site.

### [Effects of the invention]

The present invention is advantageous in that the surface of a patient's hard bone is subjected to registration in the navigation system, so that accurate alignment can be performed, and no burden for surgery intended for registration is imposed on the patient. Moreover, photographic data from single tomography can be utilized through template production, registration and navigation. This brings the advantage that it is not necessary, for example, to photograph images of the patient to whom markers were adhered in advance. This is also advantageous in that since the number of tomography operations is reduced, the number of exposures to radiation by CT is decreased, and the time required before surgery can be shortened markedly.

Furthermore, the operation of registration of the three or more registration points may be such that the registration operation in the operating room is the operation of only registration of the reflecting balls for three-dimensional position detection, and can be performed with great ease and in a short time, because the coordinates of these registration points relative to the reflecting balls are recognizedbeforehand outside the operating room. In addition, the accurate fitting of the registration template onto the surface of the patient's hard bone can be easily perceived by a sensation in the operator' s fingertips. Thus, if the posture of the patient is to be changed, or even when the posture has changed unexpectedly, during surgery, registration can be performed again easily and promptly.

### Brief Description of the Drawings

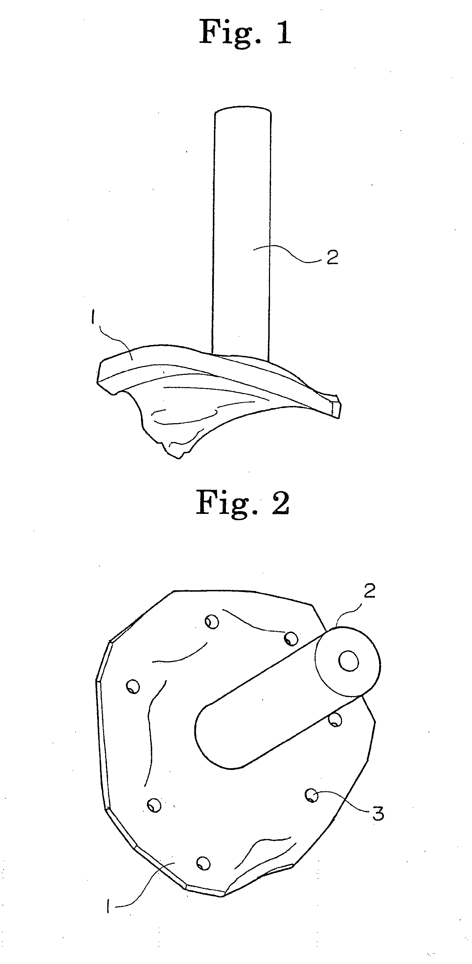

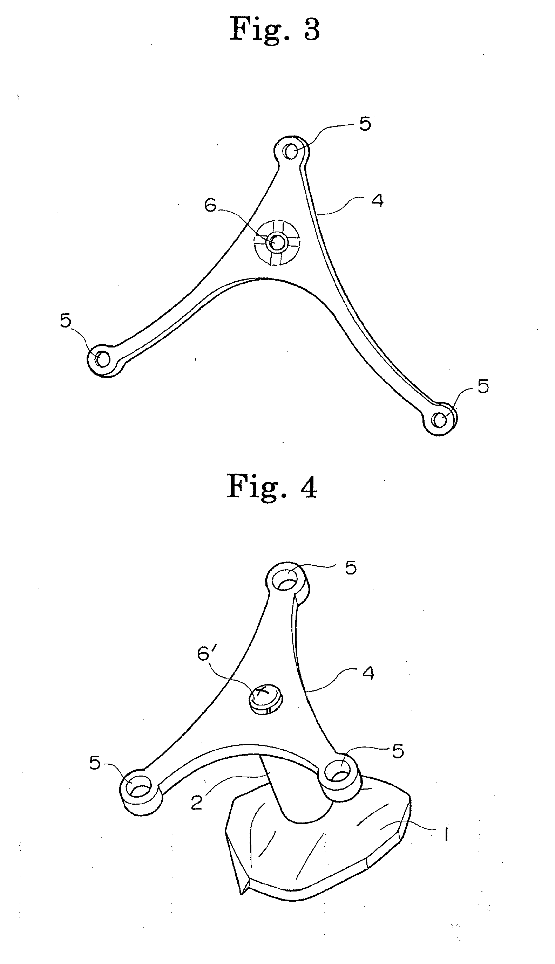

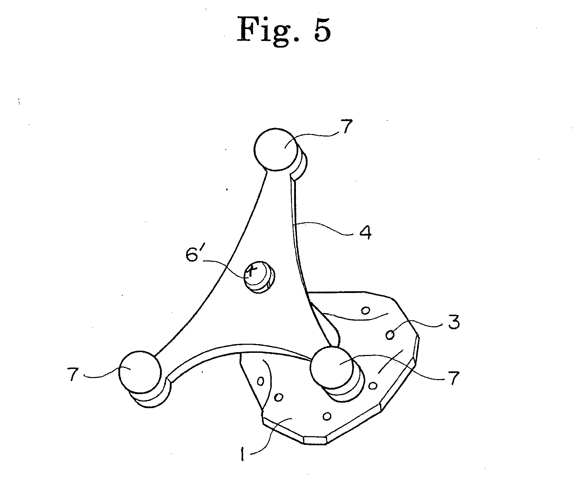

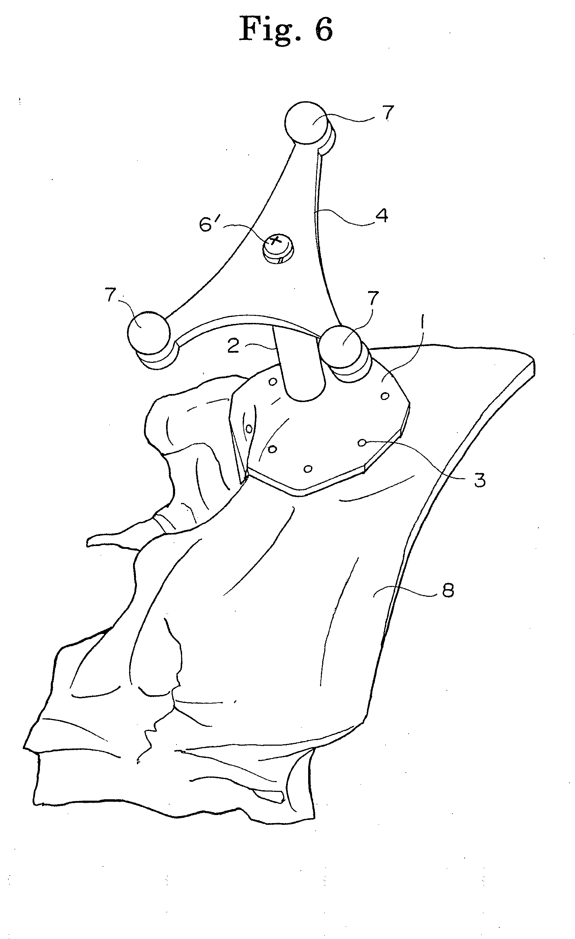

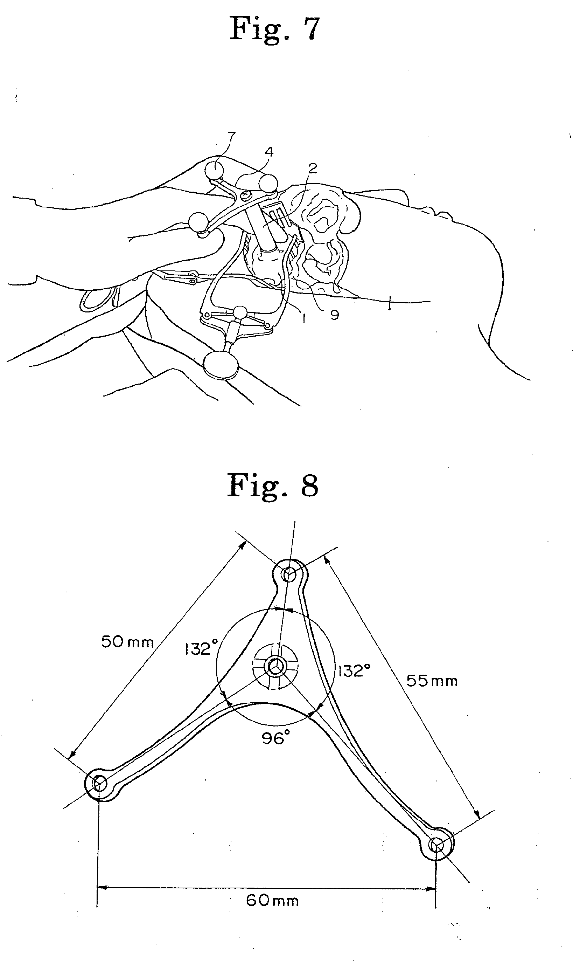

Fig. 1 is a perspective view, from a lateral direction, of an example of a registration template with a pedestal mounting pole which has been obtained by integral forming in accordance with the manufacturing method of the present invention. Fig. 2 is a perspective view, from an upper direction, of an example of the registration template with the pedestal mounting pole which has been obtained by integral forming in accordance with the manufacturing method of the present invention. Fig. 3 is a top view of an example of a pedestal used for mounting on the pole of the registration template with the pedestal mounting pole which has been obtained by integral forming in accordance with the manufacturing method of the present invention. Fig. 4 is a perspective view showing an example of a state in which the pedestal has been mounted on the pole of the registration template with the pedestal mounting pole obtained by integral forming, in accordance with the manufacturing method of the present invention. Fig. 5 is a perspective view showing an example of a state in which the pedestal has been mounted on the pole of the registration template with the pedestal mounting pole obtained by integral forming, and optical tracking balls have been further mounted on the pedestal, in accordance with the manufacturing method of the present invention. Fig. 6 is a perspective view showing a state in which the registration template equipped with an optical tracking antenna in the present invention has been bonded to the surface of a temporal site model. Fig. 7 is a perspective view showing an example of a state in which the registration template having the optical tracking antenna has been fitted onto the exposed bone surface of a temporal site of a patient in surgery of the temporal site in Example 1. Fig. 8 is a top view showing the shape and dimensions of the pedestal used in the registration template having the optical tracking antenna produced in Example 1. In these drawings, the numeral 1 denotes a site of the registration template which surface-bonds to the surface of a bone, 2 denotes the pedestal mounting pole, 3 denotes a registration point (guide hole), 4 denotes the pedestal, 5 denotes a fixing portion for the optical tracking ball, 6 denotes a site for mounting the pedestal on the pole using a bolt or a screw, 6' denotes a state in which the pedestal has been mounted on the pole using the bolt, screw or the like, 7 denotes the optical tracking ball, 8 denotes the temporal site model, and 9 denotes the exposed bone surface of the temporal site of the patient.

### Mode for Carrying Out the Invention

The method for manufacturing a registration template according to the present invention is a method for manufacturing a registration template having three or more registration points for use in medical navigation system-guided surgery. The registration template having three or more registration points obtained by this method is required to have at least three registration points in order to navigate the position of a surgical tool three-dimensionally.

The registration template is also required to have a pedestal mounting portion, conformed to the size of an optical tracking antenna in a medical navigation system, which is commercially available, at the center of these registration points in order to enable a reflection type three-dimensional position detector to be mounted thereon.

As the material for the registration template obtained by the present invention, any material can be used without limitation, as long as it has moderate hardness and has biocompatibility. For example, synthetic resin, rubber, an inorganic material, or a composite material composed of an inorganic powder and resin is sterilized, and then can be used in the field of surgical operation.

Tomography information on the bone at a surgical target site of a patient according to the present invention can be obtained based on three-dimensional image data created by extracting a region at issue based on threshold values from slice images in CT, MRI or the like and making interpolation calculations.

The manufacturing method of the present invention involves acquiring stereoscopic shape data on the surface of the bone at the surgical target site of the patient relevant to the above image data based on the stereoscopic surface data on the surface of the bone; and manufacturing a registration template having a surface precisely conformed to the curved surface of the shape data.

The method for manufacturing the registration template of the present invention has three aspects.

A first of the aspects is adapted to duplicate three-dimensional stereoscopic surface data on the bone at the surgical target site of the patient on software; displace the duplicated data by a predetermined distance in a direction outward of the bone surface to create the original stereoscopic surface data and convex or concave shape data based on position information on three or more predetermined registration points; perform data processing for subtracting these data from the displaced duplicated surface data to create stereoscopic template data which has uneven surfaces of the same shape as the shape of uneven surfaces of the bone at the surgical target site on a surface opposite to a surface precisely surface-bonding to the surface of the bone at the surgical target site and which has a predetermined wall thickness added to the surface opposite to the bone surface; impart, to the stereoscopic template data, shape data on a mounting portion for a pedestal for mounting a three-dimensional position detecting reflector for a navigation system; and produce a registration template directly by a production method, such as stereoscopic shaping, including stereolithography, inkjet printing, powder shaping, powder sintering, sheet lamination, or selective laser sintering, cutting or casting, in correspondence with the resulting stereoscopic template data.

Using any of the resulting templates as a master, a mold can be produced. Using the mold, a registration template can be manufactured by casting. Examples of a material usable for the master are polyethylene resin, silicone rubber, urethane rubber, gypsum, an alginate templating material, and polymer clay.

According to this aspect, the registration template can be manufactured directly without producing an actual-size model of the diseased part of the patient.

A second of the aspects is adapted to produce a stereoscopic actual-size model of a surgical target site of a patient by stereoscopic shaping such as, for example, stereolithography, inkjet printing, powder shaping, powder sintering, sheet lamination, or selective laser sintering, using three-dimensional stereoscopic surface data on a bone at the surgical target site of the patient, and convex or concave shape data based on position information on three or more predetermined registration points; pressing a surface of the model at a predetermined position corresponding to the surgical target site, as an embossing die, against a mold material of predetermined dimensions to form a master for production of a mold; produce a template surface, to which a surface shape of the model at the predetermined position has been transferred, by casting; and impart shape data on a mounting portion for a pedestal for mounting a three-dimensional position detecting reflector for a navigation system, thereby manufacturing a registration template. As the mold material, not only gypsum, but polyethylene resin, silicone rubber, urethane rubber, an alginate templating material, or polymer clay can also be used.

A third of the aspects is adapted to produce a stereoscopic actual-size model of a surgical target site of a patient by stereoscopic shaping such as, for example, stereolithography, inkjet printing, powder shaping, powder sintering, sheet lamination, or selective laser sintering, using three-dimensional stereoscopic surface data on a bone at the surgical target site of the patient, and convex or concave shape data based on position information on three or more predetermined registration points; obtain stereoscopic surface data ensuring precise surface bonding to a surface of the model, corresponding to the surface of the bone at the surgical target site, by a noncontact laser scanning device; form the resulting stereoscopic surface data into a plate shape of a template by use of editing software; combine the plate shape with the position information on the three or more predetermined registration points to impart shape data on a mounting portion for a pedestal for mounting a three-dimensional position detecting reflector for a navigation system; and manufacture a registration template having a surface precisely surface-bonding to the surface of the bone at the surgical target site by stereoscopic shaping. As the editing software, commercially available medical slice image editing software, for example, "Mimics" (a product of Materialize, Belgium), "3D Doctor" (Able Software Corp. , U.S.A.), or "ZedView" (LEXI Co., Ltd., Japan) can be used.

The model of the surgical target site manufactured by any of the above-described methods is the actual-size model of the diseased part of the patient. As shown in Figs. 6 and 7, the above model makes it possible to confirm the bonding position, the bonding state, and the bonding accuracy of the registration template during surgery, and can be effectively used for pre-investigation of the surgical procedures or as a training model for surgery.

The registration template of the present invention is not limited in thickness, as long as it reserves a thickness necessary for mounting a pedestal, which is used to mount optical tracking balls for use in three-dimensional position detection for a medical navigation system, at a location corresponding to the center of the template. If formed as thinly as possible, with the required strength being retained, however, the registration template can be slid in below the skin and other soft tissue of the patient, namely, over the surface of the bone, during surgery, whereby the range of incision can be minimized. From this point of view, it is desirable that a mounting portion for the pedestal for mounting the optical tracking balls, the mounting portion of a shape protuberant relative to surrounding parts be provided at the location corresponding to the center of the registration template of the present invention. The shape of the pedestal mounting portion is not limited, but is preferably columnar, and is more preferably cylindrical (pole-shaped) because of operability during surgery. The height of the pedestal mounting portion protruding from the surface precisely surface-bonding to the surface of the bone at the surgical target site is not limited, but is preferably 15 mm or more, more preferably 20 mm or more, but 70 mm or less, and particularly preferably 35 mm or more, but 55 mm or less.

Besides, the surface shape of the bone at the surgical target site is formed in at least a part of a surface opposite to the surface precisely surface-bonding to the surface of the bone at the surgical target site, the part other than the mounting portion for the pedestal for mounting the optical tracking balls. By so doing, the aforementioned first aspect, according to which the bonding position of the surface bonding can be easily judged, is simplified in terms of the manufacturing process, and the desired effects of shortening the time required for registration and lightening a physical burden on the patient are obtained.

The three or more registration points of the registration template according to the present invention are points which enable recognition of the relative positional relationship with the device, which detects three-dimensional position information on the surgical tool, in registering the specific position of the surgical site on the patient' s body and images of the navigation system in correspondence with each other during registration operation.

As the registration points in the present invention, any obj ects can be used without limitation, if they serve as markings in the registration procedure. Their examples are planar marks as landmarks for the registration template surface, colored stamps, embossedmarks, guide holes, and marks for drive-fitting of screws. The embossed marks and guide holes, in particular, are preferred, because they make stereoscopic positioning confirmable.

### [Concrete method for manufacturing registration template]

A concrete method for manufacturing the registration template of the present invention involves, as a first step, photographing of CT/MRI data on the patient for the purpose of manufacturing the registration template. The data obtained by photography during medical examination can be used.

In manufacturing the registration template of the present invention, there is no need to attach markers, intended for registration during surgery, at the time of tomography of the bone at the surgical target site. Photographic data obtained during medical examination can be used.

In the manufacturing method of the present invention, the above tomography information can be loaded into a computer using the general-purpose software "Mimics".

Using the general-purpose software "Mimics", three-dimensional image processing and editing can be performed. That is, slice images from a CT scanner or MRI are loaded in, whereby the region concerned can be extracted and three-dimensional shape data can be created. The display can show the appearance of the surgical target site viewed from an arbitrary angle and three-dimensional images of the internal structure.

For image processing in the present invention, a standard format called DICOM (Digital Imaging and Communication in Medicine) data can be used.

By using the DICOM data, the region concerned which is the site to be rendered stereoscopic can be extracted from the photographic data by use of threshold values and various segmentation functions possessed by "Mimics". The surgical target region is subjected to three-dimensional computation to obtain shape information on the bone surface in the surgical target region. Further, the shape information is duplicated, and moved by a constant amount corresponding to the wall thickness of the template in the direction of a surface opposite to the surface which bonds to the bone surface. From the resulting data, the shape information on the bone surface in the surgical target region is subtracted, whereby it becomes possible to obtain image information on the template of a predetermined wall thickness having the inner surface corresponding to the target site. Furthermore, a cylindrical shape as a pedestal mounting portion can be formed on the design screen by "Mimics".

The so formed cylindrical shape is moved to the center of the registration points, and can be used as a mounting portion for a pedestal in conformity with the size of an optical tracking antenna in a medical navigation system.

Moreover, the surgical target region is subjected to three-dimensional computation to obtain surface data on the bone surface in the surgical target region. The surface data is subtracted from shape data as box data, so that shape data on the template of a predetermined wall thickness having the inner surface of the target site can be obtained.

Based on the shape data on the template with a predetermined wall thickness, a predetermined area and predetermined dimensions having the inner surface of the target site, which have been obtained based on three-dimensional shape information on the bone surface in the surgical target region, the registration template of the present invention can be manufactured by various shaping techniques.

Because of the registration template provided with the pedestal for mounting the three-dimensional position information detecting reflector in the medical navigation system according to the present invention, information on the relative positions of the three-dimensional position detecting reflector and the three-dimensional coordinates of the three or more registration points indicated by embossed markings or guide holes set beforehand as the registration points for accurate registration of the surgical tool can be stored accurately in the medical navigation system. This relative position information is called up from the navigation system during surgery, and used as data for registration operation, whereby the registration operating time can be shortened further.

By means of the "Mimics", moreover, shape data on the three or more registration points indicated by the embossed markings or guide holes prepared on three-dimensional images can be imparted to the DICOM data on the surgical target site of the patient accurate to 0.01 mm, and outputted therefrom.

In the surgical navigation system, the relative positional relationship between the three-dimensional position information on the three or more registration points and the optical tracking balls for three-dimensional position detection used in the medical navigation system is registered beforehand, as coordinates, into the medical navigation system prior to registration. In the operating room, only registration of the optical tracking balls is performed. As a result, three-dimensional position information on the three or more registration points on the registration template can be acquired by computation within the system. Thus, the time required for registration in the operating room can be shortened markedly. These advantages contribute greatly to shortening the surgery time, and leading to a reduction in a burden on the patient in surgery.

According to the surgical navigation system, moreover, the DICOM data accurately given the shape data on pins for three or more registration points indicated by the embossed markings or guide holes, which have been outputted from the "Mimics", are loaded in. Then, registration can be performed easily and highly precisely between the three or more registration points on the DICOM and the three or more registration points on the registration template precisely surface-bonding directly to the surgical target site of the patient.

No limitations are imposed on the method for manufacturing, based on stereoscopic shape information, the registration template having predetermined wall thickness, area and dimensions in the present invention. However, a manufacturing method, such as stereoscopic shaping, including stereolithography, inkjet printing, powder shaping, powder sintering, sheet lamination, or selective laser sintering, cutting, or casting, can be used preferably.

Particularly in preparing a model of the surgical target site of the patient, together with the registration template, there may be employed the selective laser sintering method using a thermoplastic resin powder mixed with an inorganic material as a shaping material (see Japanese Patent No. 3927487), or the stereolithography process (see Japanese Unexamined Patent Publication JP-A-2010-264213). Manufacture of the model by any such method is preferred, because an actual-size model of a stereoscopic shape having the same hardness as the natural bone of the human body or the like can be duplicated three-dimensionally with precision and accuracy, and this model can be used for preoperative simulation or the like.

If a transparent material such as resin is used as a starting material in manufacturing the registration template of the present invention by stereolithography or selective laser sintering, a transparent registration template can be manufactured. This transparent template is advantageous in that the state of the bone surface to be fitted therewith can be visually recognized with ease at the time of registration.

The three-dimensional shape data used in the stereolithography method or the selective laser sintering method are used in producing a stereoscopic precision model of the living body, and obtained by perspectively measuring or externally measuring the shapes of respective sites of the human body, as the original, by one of, or a combination of two or more of, magnetic resonance imaging (MRI), X-ray computed tomography (X-ray CT), and ultrasonography. The resulting image data on the respective sites by actual measurement of the human body are once converted into two-dimensional DICOM data, for example. These two-dimensional slice images are stacked, with the origins of the respective images in alignment, and then loaded in. The spaces between the images are interpolated to form a stereoscopic image, which is in turn converted into surface type STL format data, and thereby can be applied to stereolithography, powder sintering or the like.

In order to manufacture the registration template of the present invention, the surface type STL format data created by the above data processing can be used.

In the present invention, a photosetting resin used as a material for the registration template by stereolithography is not limited and, for example, publicly known photosetting resins, such as acrylate resins or epoxy resins, can be used. Nor are any limitations imposed on a synthetic resin powder for use as a material for the registration template by selective laser sintering. Its examples are nylon, polycarbonate, polyester, polyacetal, polyethylene, polypropylene, polyvinyl chloride, polystyrene, polybutylene, ABS resin, cellulosic resin, acrylic resin, epoxy resin, andfluoroplastic. Of these materials, nylon can be used preferably, and nylon 11 can be used more preferably.

The use of a transparent material as a material for the registration template is particularly preferred during operation, because when the template is brought into intimate contact with the patient' s bone for the purpose of registration, the bone is visible.

The registration template in the present invention can be sterilized, and as a sterilization treatment, gas sterilization or coating, for example, can be applied to the template.

Next, a concrete manufacturing method for a registration template equipped with a three-dimensional position detecting reflector (may be called an optical tracking antenna) will be explained step by step.

First of all, stereoscopic surface image data is obtained on a surface precisely surface-bonding to the surface of a bone at a surgical target site in a patient, based on three-dimensional image data created from tomography information the bone at the surgical target site of the patient. From the stereoscopic surface image data, a registration template, which has the surface precisely surface-bonding to the surface of the bone at the surgical target site, which is provided with three or more registration points, and which has a pedestal mounting pole, as a pedestal mounting portion in the present invention, at nearly the center of the registration points, is integrally shaped by stereolithography or the like using the aforementioned shaping material, preferably a transparent material.

Fig. 1 and Fig. 2 are, respectively, a perspective view from a lateral direction, and a perspective view from an upper direction, of an example of a registration template with a pedestal mounting pole which has been obtained by integral forming in accordance with the manufacturing method of the present invention.

The numeral 1 denotes a site precisely surface-bonding to the surface of a bone, 2 denotes a pedestal mounting pole, and 3 denotes a registration point (an embossed mark or a guide hole).

The length of the pole is normally of the order of 5 to 50 mm, and its diameter is normally of the order of 8 to 12 mm.

Then, a pedestal for fixing optical tracking balls is mounted on the pole of the thus obtained registration template with the pedestal mounting pole.

Fig. 3 is a top view of an example of a pedestal used for mounting on the pole of the registration template with the pedestal mounting pole which has been obtained by integral forming in accordance with the above-described method. Fig. 4 is a perspective view showing an example of a state in which the pedestal has been mounted on the pole of the registration template with the pedestal mounting pole obtained by integral forming.

In Fig. 3, the numeral 4 denotes the pedestal, 5 denotes a fixing portion for the optical tracking ball, and 6 denotes a site for mounting the pedestal 4 on the pole using a bolt or a screw. In Fig. 4, the numerals 1, 2, 4 and 5 are as mentioned above, and 6' denotes a state in which the pedestal 4 has been mounted on the pole 2 by means of a bolt, screw or the like.

Then, optical tracking balls 7 are mounted on the optical tracking ball fixing portions 5 of the pedestal mounted on the pole of the registration template with the pedestal mounting pole obtained by integral forming to produce a registration template having a three-dimensional position detecting reflector (optical tracking antenna). That is, the pedestal 4 having the optical tracking balls 7 fixed thereto functions as the optical tracking antenna.

Fig. 5 is a perspective view showing an example of a state in which the pedestal has been mounted on the pole of the registration template with the pedestal mounting pole obtained by integral forming, and the optical tracking balls have been mounted on the pedestal, in accordance with the manufacturing method of the present invention.

In Fig. 5, the numerals 1, 3, 4 and 6' are as described above, and 7 denotes the optical tracking ball.

Fig. 6 is a perspective view showing a state in which the registration template equipped with the optical tracking antenna in the present invention has been bonded to the surface of a temporal site model. The numerals 1, 2, 3, 4, 6' and 7 are as described above, and 8 denotes the temporal site model.

The tracking ball 7 is usually a plastic ball coated with an infrared reflective sheet, and has a diameter of the order of 7 to 13 mm.

### Example

Next, the present invention will be described in further detail by its working example. However, the present invention is in no way limited by this example.

### Example 1

Amodel of a temporal site as a surgical target was produced by powder sintering, and a registration template intimately contacting the temporal site was manufactured by stereolithography.

A mixture of 70% by mass of a spherical nylon 11 powder having an average particle diameter of 58 µm and 30% by mass of glass beads having an average particle diameter of 60 µm was used as a material to be powder-sintered, and a selective laser sintering device with a 100W carbon dioxide laser ["Vanguard-HS", a product of 3D-Systems] was used as a shaping device. An epoxy resin (trade name TSR-829, produced by CMET Inc.) was used as a stereolithographic material, and the shaping device ("SOLIFORM600EP", produced by CMET Inc.) was used.

Based on data from X-ray computed tomography of a bone at a temporal site in a child, data on the temporal site and a registration template were created. To the data on the template, data on the positions and dimensions of eight guide holes were added.

Using the software "Mimics" (produced by Materialise), these data were converted into shaping data. The data was inputted to the shaping device, by which the powder sintering material was sequentially laminated and sintered at a lamination pitch of 0. 10 mm to produce an actual-size temporal site model. The stereolithographic material was sequentially laminated and shaped at a lamination pitch of 0.125 mm using a stereolithography device to integrally produce a registration template having eight guide holes 3 with the dimensions 43x38 mm and a thickness of 3 mm and having at the center thereof a pedestal mounting pole with a diameter of 10 mm and a length of 40 mm as shown in Fig. 2. A pedestal having the shape shown in the top view of Fig. 3 (its dimensions are shown in the top view of Fig. 8) was attached to the pole using a screw at the site of the numeral 6. Then, the optical tracking balls 7 were fixed to the optical tracking ball fixing portions 5 of the pedestal with the use of an adhesive to manufacture a registration template equipped with an optical tracking antenna.

As shown in Fig. 6, when the registration template shown in Fig. 5 was brought into contact with the surface of the produced temporal site model 8, their surfaces bonded to each other with high precision, and the resulting bond was so stable that they did not move even when they were swung longitudinally and laterally.

In such a stable bonding state, it was possible to perform registration while applying a probe, with the guide holes 3 as the registration points. It was also possible to place colored marks, as registration points, on the surface of the temporal site model 8 shown in Fig. 6 via the guide holes 3 shown in Fig. 6.

In performing surgery, moreover, before the patient entered the operating room, the spatial coordinates of these registration points were acquired optically, and these spatial coordinates were stored as the coordinates relative to the optical tracking balls 7. After sterilization of the registration template, surgery on the patient was started in the operating room. As shown in Fig. 7, the bone surface was exposed, and the registration template 1 fitted with the optical tracking balls 7 was applied to a location of the bone surface unique to surface bonding to obtain the coordinates of the optical tracking balls 7. From the spatial coordinates of the balls, the coordinates of the registration points on the registration template were calculated backwards, and successfully aligned with the CT coordinates within the computer of the navigation system.

Fig. 7 is a perspective view showing an example of a state in which the registration template having the optical tracking antenna has been fitted onto the exposed bone surface of the temporal site of the patient in surgery of the temporal site in the present Example. The numeral 9 denotes the exposed bone surface of the temporal site of the patient.

The work time that the operator required for registration in the operating room was 30 seconds or less from the installation of the registration template with the optical tracking balls subsequently to the exposure of the bone surface until completion of registration. This procedure markedly shortened the work time as compared with the conventional point registration requiring 10 minutes or so on the average.

### Industrial Applicability

The registration template of the present invention can markedly decrease work for registration after entry into the operating room, and lessens a burden on the patient who undergoes surgery. In surgery using a medical navigation system, alignment can be performed based on the patient's bone. A single registration operation is enough to achieve registration having precision comparable to the precision of registration, which is performed directly in the operating room, by use of three or more registration points. Moreover, alignment between images and the surgical target site of the patient can be performed with high precision. Furthermore, registration can be performed promptly in response to changes in the posture of the patient during surgery. Hence, the registration template of the present invention is very useful.

## Claims

1. A method for manufacturing a registration template having three or more registration points for use in medical navigation system-guided surgery, comprising:
producing a template having a surface, which precisely surface-bonds to a surface of a bone at a surgical target site of a patient, by a production method selected from among stereoscopic shaping, cutting, and casting, from stereoscopic surface data ensuring precise surface bonding to the surface of the bone at the surgical target site of the patient, based on stereoscopic surface data created from tomography information on the bone at the surgical target site of the patient; and
providing a pedestal for mounting optical tracking balls, which are used for three-dimensional position detection for a medical navigation system, at a center of the registration points of the template.

2. The method for manufacturing a registration template according to claim 1, further comprising:
producing a template having a mounting portion for the pedestal for mounting the optical tracking balls, which are used for three-dimensional position detection for the medical navigation system, at a location corresponding to the center of the three or more registration points for use in the medical navigation system, the location being on a surface on a side opposite to the surface precisely surface-bonding to the surface of the bone at the surgical target site; and
providing the pedestal for mounting the optical tracking balls, on the mounting portion for the pedestal for mounting the optical tracking balls.

3. The method for manufacturing a registration template according to claim 2, wherein
the mounting portion for the pedestal for mounting the optical tracking balls has a columnar shape.

4. The method for manufacturing a registration template according to any one of claims 1 to 3, wherein
the registration points are embossed marks.

5. The method for manufacturing a registration template according to any one of claims 1 to 3, wherein
the registration points are guide holes.

6. The method for manufacturing a registration template according to any one of claims 1 to 5, further comprising
producing the template, which has the surface precisely surface-bonding to the surface of the bone at the surgical target site, by stereolithography.

7. The method for manufacturing a registration template according to any one of claims 1 to 6, further comprising:
producing the template having the surface, which precisely surface-bonds to the surface of the bone at the surgical target site of the patient, from the stereoscopic surface data ensuring precise surface bonding to the surface of the bone at the surgical target site of the patient, based on the stereoscopic surface data created from the tomography information on the bone at the surgical target site of the patient; and
processing the data to produce a model of the surgical target site of the patient.

8. A method for manufacturing a registration template having three or more registration points for use in medical navigation system-guided surgery, and having a surface precisely surface-bonding to a surface of a bone at a surgical target site, comprising:
producing a template, in which the surface precisely surface-bonding to the surface of the bone at the surgical target site has not been formed, but a mounting portion for a pedestal for mounting optical tracking balls for use in three-dimensional position detection for a medical navigation system has been provided at the center of the registration points of the template, by a production method selected from stereoscopic shaping and cutting;
producing a model of the surgical target site based on three-dimensional data created from tomography information on the bone at the surgical target site of a patient and position information on three or more predetermined registration points;
pressing the surface of the bone at the surgical target site in the model, as an embossing die, against a mold material of predetermined dimensions, and pressing the mold material having a surface shape of the model of the surgical target site against a surface of the template, in which the surface precisely surface-bonding to the surface of the bone at the surgical target site has not been formed and is to be formed, to transfer the surface shape of the model of the surgical target site to the surface of the template, thereby producing the template having the surface shape transferred thereto;
producing a mold by use of the resulting template as a mold producing master; and
producing the template by casting using the mold.

9. A method for manufacturing a registration template having three or more registration points for use in medical navigation system-guided surgery, and having a surface precisely surface-bonding to a surface of a bone at a surgical target site, comprising:
producing a model of a surgical target site of a patient by stereoscopic shaping based on three-dimensional image data created from tomography information on the surgical target site of the patient;
scanning a surface of a site in the model, corresponding to the surgical target site, by a noncontact laser scanning device to obtain stereoscopic surface data ensuring precise surface bonding to the surface of the site in the model corresponding to the surgical target site;
loading the resulting stereoscopic surface data onto editing software;
duplicating and moving the stereoscopic surface data loaded onto the editing software for conversion into a plate form;
imparting data on three or more predetermined registration points to the plate form; and
further providing a mounting portion for a pedestal for mounting optical tracking balls for use in three-dimensional position detection for a medical navigation system, at the center of the plate form by use of a shape generating function of the editing software, thereby producing a template having a surface precisely surface-bonding to the surface of a bone at the surgical target site.

10. The method for manufacturing a registration template according to any one of claims 1 to 9, wherein
the registration template is transparent.

11. The method for manufacturing a registration template according to any one of claims 1 to 10, wherein
the registration template has at least some of concave and convex surfaces of a shape identical with a shape of concave and convex surfaces of the bone at the surgical target site, on a surface opposite to the surface precisely surface-bonding to the surface of the bone at the surgical target site.
